# EUROPÄISCHE PATENTSCHRIFT

(11) **EP 0 978 271 B1**
(45) Veröffentlichungstag und Bekanntmachung des Hinweises auf die Patenterteilung: **23.05.2001**
(21) Anmeldenummer: 99114295.1
(22) Anmeldetag: 29.07.1999
(51) Int. Cl.: A61K 7/06

(54) **Aerosolschaumzusammensetzung**
Aerosol foam composition
Composition pour mousse aérosol

(30) Priorität: 03.08.1998 DE 19834946
(43) Veröffentlichungstag der Anmeldung: 09.02.2000
(73) Patentinhaber: GOLDWELL GmbH, 64297 Darmstadt (DE)
(72) Erfinder: Üllner, Martin, 64295 Darmstadt (DE); Cajan, Christiane, 56130 Bad Ems (DE)

(56) Entgegenhaltungen:
- EP-A- 0 172 713
- EP-A- 0 213 827
- EP-A- 0 445 714

## Beschreibung

Die vorliegende Erfindung betrifft eine Aerosolschaumzusammensetzung, insbesondere zur Aufbringung auf menschliches Haar.

Derartige Zusammensetzungen sind an sich bekannt und werden insbesondere als Haarkonditioniermittel, sogenannte "Styling foams", eingesetzt.

Solche Zusammensetzungen enthalten, in wäßriger Grundlage, üblicherweise haarpflegende und -konditionierende Stoffe oder Filmbildner, gegebenenfalls Tenside, Treibmittel und alle möglichen gewünschten Zusatzstoffe, z.B. direktziehende Haarfarbstoffe, um einen Haarfärbeeffekt zu bewirken.

Es besteht jedoch auch der Wunsch nach Aerosolschaumzusammensetzungen, die auf dem Haar andere optische Effekte, wie einen besonderen Glanz, z.B. Perlglanz, hervorrufen.

Die Realisierung derartiger Wünsche scheiterte bisher jedoch daran, daß es nicht möglich war, entsprechende Glanzpigmente so in eine Grundlage einzuarbeiten, daß sie im Produkt nicht ausfallen, verklumpen und so das Ventil verstopfen und damit die erwünschte Glanzwirkung im Produkt und auf dem Haar nicht entfalten können, sondern die Produkteigenschaften insgesamt negativ beeinflussen.

Es wurde nunmehr gefunden, daß sich dieses Problem dadurch lösen läßt und eine stabile Aerosolschaumzusammensetzung mit glänzendem Aussehen und beim Aufbringen auf ein Substrat, insbesondere menschliches Haar, diesem Glanz verleihenden Eigenschaften erhalten werden kann, wenn diese Zusammensetzung in wäßriger Grundlage ein Gemisch aus mindestens einem Glanzpigment, mindestens einem Aerosoltreibmittel und einem (Meth)Acrylsäure-Polymerisat enthält.

Diese Mittel zeichnen sich nicht nur durch die obengenannten Eigenschaften, sondern auch durch eine hervorragende Stabilität im Behältnis aus, was insbesondere dann, wenn es sich um ein transparentes Glas- oder Kunststoffbehältnis handelt, zu einem attraktiven, ästhetisch ansprechenden Produkt mit gleichmäßig verteilten Glanzpigmenten führt.

Außerdem läßt sich der bei der Abgabe des Produktes erhaltene Schaum gut verteilen und zerfällt rasch.

Als bevorzugte Glanzpigmente, die vorzugsweise in einer Menge von etwa 0,05 bis 2,5, vorzugsweise 0,1 bis 1,5 Gew.-%, berechnet auf die Gesamtzusammensetzung des erfindungsgemäßen Mittels, anwesend sind, werden Glimmer-Pigmente, insbesondere mit Metalloxiden beschichtete Glimmer-Pigmente eingesetzt, wie sie beispielsweise unter dem Stichwort "Mica" im "CTFA International Cosmetic Ingredient Dictionary" beschrieben sind. Geeignete Metalloxide, die auch Farbstoffe enthalten können, sind insbesondere Titanoxid und durch Verwendung von Wismutoxidchlorid entstandene Wismutoxide; geeignet sind auch Wismutoxidchlorid selbst und Wismutoxidchlorid-Talk-Pigrnente sowie natürliches Fischsilber.

Der bevorzugte Teilchendurchmesser dieser Glanzpigmente liegt bei etwa 1 bis etwa 200, insbesondere etwa 10 bis etwa 150 µm.

Der zweite essentielle Bestandteil der erfindungsgemäßen Zusammensetzung ist ein gegebenenfalls vernetztes Acrylsäure- oder Methacrylsäure-Polymerisat. Vorzugsweise handelt es sich dabei um Copolymerisate aus (Meth)Acrylsäure und höheren Alkylestern der Acrylsäure und Methacrylsäure. wie C₈-C₃₂-, insbesondere C₁₀-C₃₀-Alkyl(meth)-acrylaten.

Geeignete Produkte sind unter der CTFA-Bezeichnung "Acrylates Copolymer" bekannt, insbesondere "Acrylates C10-C30-Alkyl Acrylates Crosspolymer", beispielsweise "Carbopol® ETD 2020", "Carbopol® 1342", Pemulen® TR-1" und "TR-2", sowie Acrylsäure/Stearyl- bzw. Cetylmethacrylat- und Acrylsäure/Steareth-20- bzw. Ceteth-20-methacrylat-Copolymere.

Der Anteil dieser (Meth)Acrylsäure-Polymeren in den erfindungsgemäßen Produkten liegt vorzugsweise bei etwa 0,05 bis etwa 2,5, insbesondere etwa 0,1 bis etwa 1,5 Gew.-%, berechnet auf die Gesamtzusammensetzung.

Der dritte essentielle Bestandteil, mindestens ein Treibmittel, ist in den erfindungsgemäßen Zusammensetzungen in einer Menge von vorzugsweise etwa 5 bis 25, insbesondere etwa 10 bis 20 Gew.-%, berechnet auf die Gesamtzusammensetzung, enthalten.

Bevorzugtes Treibmittel ist Dimethylether, bei dessen Verwendung ein Schaum erhalten wird, der beim Verreiben auf der Handfläche einen deutlichen Kühleffekt bewirkt.

Weitere geeignete Treibmittel sind Kohlenwasserstoffe wie Propan, n-Butan und Isobutan sowie deren Gemische.

Auch die Verwendung von Kohlendioxid als Treibmittel ist möglich.

Je nach Bedarf können der Zusammensetzung noch Lösungsmittel wie niedere Mono- und Polyalkohole, insbesondere Alkandiole und Glycerin sowie Kohlenwasserstoffe wie n-Pentan, Isopentan, n-Hexan und n-Heptan zugesetzt werden.

Weiterhin enthalten die erfindungsgemäßen Mittel vorzugsweise natürliche und/oder synthetische, haarkonditionierende Polymere, insbesondere in einer Menge von 0,05 bis 5, bevorzugt 0,1 bis 2,5 Gew.-% der Gesamtzusammensetzung.

Als geeignete kationische Polymere sind neben den altbekannten quaternären Cellulosederivaten des Typs "Polymer JR" insbesondere quaternisierte Homo- und Copolymere des Dimethyldiallylammoniumchlorids, wie sie unter dem Handelsnamen "Merquat®" im Handel sind, quatemäre Vinylpyrrolidon-Copolymere, insbesondere mit Dialkylaminoalkyl(meth)acrylaten, wie sie unter dem Namen "Gafquat®" bekannt sind, Copolymerisate aus Vinylpyrrolidon und Vinylimidazoliniummethochlorid, die unter dem Handelsnamen "Luviquat® " angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniurnverbindungen der in der US-PS 4 157 388 beschriebenen Harnstoff-Struktur, die unter dem Handelsnamen "Mirapol® A 15" im Handel sind, zu nennen.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymere eingesetzt werden.

Anstelle der kationischen Polymeren oder in Kombination mit denselben können auch nichtionische Polymere verwendet werden. Als geeignete nichtionische Polymere werden vor allem Vinylpyrrolidon-Homo- und Copolymerisate, insbesondere Polyvinylpyrrolidon selbst, Copolymere aus Vinylpyrrolidon und Vinylacetat oder Terpolymerisate aus Vinylpyrrolidon, Vinylacetat und Vinylpropionat, wie sie beispielsweise von der Firma BASF unter dem Handelsnamen "Luviskol® " vertrieben werden, eingesetzt.

Es können jedoch auch (Co-)Polymerisate aus den verschiedenen Acryl- und Methacrylestern, Acrylamid und Methacrylamid, beispielsweise Polyacrylamid mit Molgewichten von über 100.000, Dimethylhydantoin-Formaldehyd-Harze, etc., verwendet werden. Selbstverständlich sind auch Mischungen aus verschiedenen nichtionischen Polymeren verwendbar.

Geeignet sind schließlich auch noch amphotere Polymere, z.B. die unter der Bezeichnung "Amphomer®" vertriebenen Copolymerisate aus N-Octylacrylamid, N-Butylaminoethylmethacrylat und Acrylsäure, sowie die bekannten anionischen Produkte, die im Stand der Technik ebenfalls hinreichend beschrieben sind.

Die erfindungsgemäßen Mittel können weiterhin die in solchen Zusammensetzungen üblichen Zusätze enthalten. Es sind dies vor allem Fette, Fettalkohole, Emulgatoren, pH-Regulatoren, kationische und anionische Farbstoffe, UV-Absorber, Lösungsmittel, Verdickungsmittel, Lösungsvermittler, Konservierungsmittel, Parfüms, etc.

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylenglykole, etc.

Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in den erfindungsgemäßen Zusammensetzungen vorzugsweise in einer Gesamtmenge von etwa 0,5 bis etwa 10, insbesondere etwa 1 bis 7,5, vor allem etwa 1,5 bis 5 Gew.-%, berechnet auf die Gesamtzusammensetzung, enthalten.

Die erfindungsgemäßen Mittel enthalten vorzugsweise auch oberflächenaktive Substanzen wie anionische, kationische, amphotere bzw. zwitterionische und insbesondere nichtionische Tenside, vorzugsweise in einer Menge von etwa 0,25 bis etwa 10, insbesondere etwa 0,5 bis etwa 5 Gew.-% des Mittels.

Eine weitere Gruppe von aktiven Ingredientien sind C₁₀-C₂₄-Fettsäuren, insbesondere Behensäure und Stearinsäure, vorzugsweise zwischen 0,1 und 10 Gew.-% der Gesamtzusammensetzung, sowie die üblichen haarkonditionierenden langkettigen quaternären Ammoniumverbindungen.

Eine Zusammenfassung der Herstellung solcher Mittel findet sich in der bereits erwähnten Monographie von K. Schrader, S. 798 bis 815, insbesondere S. 804 ff.

Die erfindungsgemäßen Mittel weisen vorzugsweise eine Viskosität (ohne Treibmittel) von mindestens 2.000 bis etwa 100.000 mPa•s bei 20 °C, gemessen im Brookfield Rotationsviskosimeter bei 10 U/min mit Spindeln Nr. 4 bzw. Nr. 5, insbesondere im Bereich zwischen etwa 5.000 und 80.000, vor allem etwa 10.000 und 50.000 mPa•s bei 20 °C auf.

Der pH-Wert der erfindungsgemäßen Mittel liegt vorzugsweise bei 3 bis 8, insbesondere zwischen 4 und 6.

Die folgenden Beispiele beschreiben Zusammensetzungen der erfindungsgemäßen Mittel.

Es wurden Haarnachbehandlungsmittel der folgenden Zusammensetzungen durch Vermischen der Bestandteile, gegebenenfalls in Form von Vormischungen, hergestellt.

### Beispiel 1

Eine Zusammensetzung aus

| | |
|---|---|
| 6,0 (Gew.-%) | Glycerin |
| 5,0 | PEG-32 |
| 0,3 | PEG-40-hydriertes Rizinusöl |
| 0,4 | Laureth-23 |
| 0,2 | Hydroxyethylcellulose |
| 0,2 | Mica (mit TiO₂ überzogen) |
| 0,2 | Acrylates /C10-C30-Alkyl Acrylates Cross Polymer |
| 0,8 | Polyquaternium-11 |
| 1,0 | Dimethicone Copolyol Methyl Ether |
| 15,0 | Isopentan |
| 0,1 | Aminomethylpropanol |
| 0,2 | Konservierungsmittel |
| 0,3 | Parfum |
| ad 100,0 | Wasser |

die eine Viskosität von 20.000 mPa•s bei 20 °C, gemessen im Brookfield-Rotationsviskosimeter bei 10 U/min mit Spindel Nr. aufwies, wurde im Gewichtsverhältnis 10:1 mit einer handelsüblichen Propan/Isobutan-Treibmittelmischung abgefüllt, wobei ein Druck von 4,2 bar erreicht wurde.

Das erhaltene Produkt wies bei der Lagerung in einer Glasflasche eine stabile Perlglanzfärbung auf; bei der Abgabe wurde ein Schaum erhalten, der rasch zerfiel und sich im Haar gleichmäßig verteilen ließ.

Der Ersatz des Acrylsäure/C₁₀-C₃₀-Alkylmethacrylat-Copolymers durch die gleiche Menge Hydroxyethylcellulose ergab eine instabile Mischung, die zum Ausfallen und Verklumpen des Glanzpigments in der Aerosol-Glasflasche führte und keine homogene Schaumabgabe erlaubte.

### Beispiel 2

Eine Zusammensetzung analog Beispiel 1, die jedoch kein Isopentan enthielt und anstelle dessen einen entsprechend erhöhten Wassergehalt aufwies, wurde im Gewichtsverhältnis 10:1 mit Dimethylether in einen Aerosolglasbehälter abgefüllt.

Es wurde ein Produkt mit einem stabilen Perlglanz erhalten, der als schnellbrechender, fein verteilbarer Aerosolschaum abgegeben wurde und zusätzlich beim Verteilen auf der Handfläche einen angenehmen Kühleffekt hervorrief.

Auf dem Haar wurde eine gute Konditionierwirkung mit gleichmäßigem Glanzeffekt erreicht.

Der Austausch des Acrylsäure-Copolymers durch die gleiche Menge Polyvinylpyrrolidon führte zu einem instabilen Produkt mit inhomogenem Schaum und einer völlig ungleichmäßigen Glanzverteilung auf dem Haar.

## Patentansprüche

1. Aerosolschaumzusammensetzung auf wäßriger Basis, enthaltend ein Gemisch aus
a) mindestens einem Glanzpigment;
b) einem gegebenenfalls vernetzten (Meth)Acrylsäure-Polymerisat; und
c) mindestens einem Aerosoltreibmittel.

2. Zusammensetzung nach Anspruch 1, enthaltend als Bestandteil b) ein (Meth)Acrylsäure/ C₈-C₃₂ Alkyl(meth)acrylat-Copolymerisat.

3. Zusammensetzung nach Anspruch 1 und/oder 2, enthaltend etwa 0,05 bis 1,5 Gew.-% des Bestandteils b), berechnet auf die Gesamtzusammensetzung.

4. Zusammensetzung nach einem oder mehreren der Ansprüche 1 bis 3, enthaltend als Bestandteil a) ein Glimmer-Pigment.

5. Zusammensetzung nach Anspruch 4, enthaltend als Bestandteil a) ein mit Metalloxiden beschichtetes Glimmer-Pigment.

6. Zusammensetzung nach einem oder mehreren der Ansprüche 1 bis 5, enthaltend den Bestandteil a) in einer Menge von etwa 0,05 bis 2,5 Gew.-%, berechnet auf die Gesamtzusammensetzung.

7. Zusammensetzung nach einem oder mehreren der Ansprüche 1 bis 6, gekennzeichnet, durch eine Viskosität von mindestens 2.000 mPa • s bei 20 °C (gemessen im Brookfield-Rotationsviskosimeter).

8. Zusammensetzung nach einem oder mehreren der Ansprüche 1 bis 7, enthaltend etwa 5 bis 25 Gew.-% des Bestandteils c).

9. Zusammensetzung nach einem oder mehreren der Ansprüche 1 bis 8, enthaltend Dimethylether als Bestandteil c).

10. Verwendung einer Zusammensetzung nach einem oder mehreren der Ansprüche 1 bis 9 zur Behandlung von menschlichen Haaren.

## Claims

1. Aerosol foam composition on aqueous basis, comprising a mixture of
a) at least one gloss pigment;
b) one optionally cross-linked (meth)acrylic acid polymer, and
c) at least one aerosol propellant.

2. Composition according to claim 1, comprising as component b) a (meth)acrylic acid/ C₈-C₃₂ alkyl(meth) acrylate copolymer.

3. Composition according to claims 1 and/or 2, comprising about 0.05% to 1.5% by weight of component b), calculated to the total composition.

4. Composition according to one or more of claims 1 to 3, comprising as component a) a glimmer pigment.

5. Composition according to claim 4, comprising as component a) a glimmer pigment coated with metal oxides.

6. Composition according to one or more of claims 1 to 5, comprising the component a) in an amount of about 0.05% to 2.5% by weight, calculated to the total composition.

7. Composition according to one or more of claims 1 to 6, having a viscosity of at least 2,000 mPa • s at 20 °C (measured in the Brookfield Rotation Viscosimeter).

8. Composition according to one or more of claims 1 to 7, comprising about 5% to 25% by weight of component c).

9. Composition according to one or more of claims 1 to 8, comprising dimethyl ether as component c).

10. Use of a composition according to one or more of claims 1 to 9 for the treatment of human hair.

## Revendications

1. Composition de mousse en aérosol à base aqueuse, comportant un mélange composé de
a) au moins un pigment brillant ;
b) un polymérisat d'acide (méth)acrylique éventuellement réticulé ;
c) au moins un propulseur pour aérosol.

2. Composition selon la revendication 1, comportant en tant que composant b) un copolymérisat d'acide (méth)acrylique/(méth)acrylate d'alkyle en C₈ à C₁₂.

3. Composition selon la revendication 1 et/ou 2, comportant environ 0,05 à 1,5 % en poids du composant b), rapporté à la composition totale.

4. Composition selon une ou plusieurs des revendications 1 à 3, comportant en tant que composant a) un pigment de mica.

5. Composition selon la revendication 4, comportant en tant que composant a) un pigment de mica revêtu d'oxydes métalliques.

6. Composition selon une ou plusieurs des revendications 1 à 5, comportant le composant a) dans une quantité comprise entre environ 0,05 et 2,5 % en poids, rapporté à la composition totale.

7. Composition selon une ou plusieurs des revendications 1 à 6, caractérisée par une viscosité d'au moins 2 000 mPa.s à 20 °C (telle que mesurée dans un viscosimètre rotatif Brookfield).

8. Composition selon une ou plusieurs des revendications 1 à 7, comportant environ 5 à 25 % en poids du composant c).

9. Composition selon une ou plusieurs des revendications 1 à 8, comportant en tant que composant c) de l'éther diméthylique.

10. Utilisation d'une composition selon une ou plusieurs des revendications 1 à 9 pour le traitement de cheveux humains.
